(19) Europäisches Patentamt
European Patent Office
Office européen des brevets

(11) **EP 2 606 815 B1**

(12) **EUROPÄISCHE PATENTSCHRIFT**

(45) Veröffentlichungstag und Bekanntmachung des Hinweises auf die Patenterteilung:
**19.03.2014 Patentblatt 2014/12**

(51) Int Cl.:
*A61B 3/08* (2006.01)         *G02B 27/22* (2006.01)
*A61B 3/032* (2006.01)

(21) Anmeldenummer: **12199154.1**

(22) Anmeldetag: **21.12.2012**

(54) **Sehprüfgerät**

Eye testing device

Appareil de contrôle visuel

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **22.12.2011 DE 102011089705**

(43) Veröffentlichungstag der Anmeldung:
**26.06.2013 Patentblatt 2013/26**

(73) Patentinhaber: **Carl Zeiss Vision International GmbH**
**73430 Aalen (DE)**

(72) Erfinder:
• **Cabeza Guillén, Jesùs-Miguel**
  **73434 Aalen (DE)**
• **Gamperling, Michael**
  **89340 Leipheim (DE)**
• **Kubitza, Matthias**
  **73431 Aalen (DE)**

(74) Vertreter: **Gauss, Nikolai et al**
**Wolf & Lutz Patentanwälte**
**Hauptmannsreute 93**
**70193 Stuttgart (DE)**

(56) Entgegenhaltungen:
**WO-A1-2008/025765      WO-A1-2009/044334**
**US-A- 5 877 840         US-A1- 2011 109 964**
**US-B1- 6 329 963**

**Beschreibung**

Die Erfindung betrifft ein Sehprüfgerät für das Untersuchen von Winkelfehlsichtigkeiten der Augen eines Probanden mit einer Bilderzeugungsvorrichtung, die ein Display für das Erzeugen von Testmustern enthält, die den Augen des Probanden mit einem optischen Strahlengang zur Anzeige gebracht werden können.

Sehprüfgeräte für das Untersuchen von Winkelfehlsichtigkeiten der Augen eines Probanden sind bekannt (z.B. EP 0 512 443 B1). Das dort beschriebene Sehprüfgerät enthält ein erstes LCD-Display und ein zweites LCD-Display. Das erste LCD-Display und das zweite LCD-Display sind hintereinander angeordnet. Das Sehprüfgerät enthält eine Lichtquelle, welche die LCD-Displays mit Durchlicht beleuchtet. An der Lichteinfallsseite eines jeden der beiden Displays ist jeweils ein farbneutraler, durchsichtiger Polarisator angeordnet. Die Polarisatoren haben Polarisationsachsen, die voneinander verschieden sind. Um mit dem Sehprüfgerät die Augen eines Probanden zu überprüfen, wird dem Proband eine Polarisationsbrille aufgesetzt, die Brillengläser mit für das rechte und linke Auge unterschiedlichen Polarisationsrichtungen hat, die den Polarisationsachsen der Polarisatoren jeweils entsprechen. Die Anzeigen der ersten und zweiten LCD-Displays werden damit aufgrund der unterschiedlichen Polarisation des Lichts getrennt. Dies hat zur Folge, dass wenn der Proband seinen Kopf zur Seite neigt, die an den beiden LCD-Displays angezeigte Information nicht mehr voneinander sauber getrennt visualisiert wird.

Ein Sehprüfgerät gemäß der Oberbegriffe der Ansprüche 1 und 4 ist aus US 5877840 bekannt.

Aufgabe der Erfindung ist es, ein Sehprüfgerät bereitzustellen, mit dem sich Winkelfehlsichtigkeiten der Augen eines Probanden untersuchen lassen und die Abhängigkeit des Seheindrucks des Probanden von der Kopfhaltung verringert ist.

Diese Aufgabe wird durch ein Sehprüfgerät mit den Merkmalen von Anspruch 1 oder den Merkmalen von Anspruch 4 gelöst. Vorteilhafte Ausführungsformen der Erfindung sind in den Unteransprüchen angegeben.

Bei einem erfindungsgemäßen Sehprüfgerät ist auf der in dem optischen Strahlengang zu den Augen des Probanden weisenden Seite des Displays eine Optikbaugruppe angeordnet, die das von einer ersten Gruppe ausgewählter Zonen des Displays dem optischen Strahlengang zugeführte Licht von dem Licht trennt, das dem Strahlengang von einer zweiten Gruppe ausgewählter Zonen des Displays zugeführt ist, um dem linken Auge des Probanden das Licht aus der ersten Gruppe ausgewählter Zonen des Displays zuzuführen und das Licht aus der zweiten Gruppe ausgewählter Zonen des Displays zu dem rechten Auge des Probanden zu leiten. Das Display in dem Sehprüfgerät kann z.B. ein LCD-Display, ein LED-Display oder ein OLED-Display sein.

Eine Idee der Erfindung besteht darin, in dem Sehprüfgerät eine Optikbaugruppe mit einer Prismenmatrix vorzusehen. Indem die Prismenmatrix eine Vielzahl von Prismenabschnitten aufweist, die in der vertikalen Richtung erstreckt sind und die jeweils einen dem Display zugewandten linsenförmigen, insbesondere zylinderlinsenförmigen Bereich mit einer konvexen Oberfläche aufweisen, können einem Probanden bei einem Sehtest unterschiedliche Testmuster mit einem auf eine bevorzugte Position des Probanden gerichteten Strahlengang visualisiert werden. Dies bietet den Vorteil, dass aus dem Seheindruck zu einem Muster, das einem Probanden im Rahmen eines Sehtests visualisiert wird, mit großer Zuverlässigkeit auf das Vorliegen oder Nichtvorliegen eines Sehfehlers geschlossen werden kann. Darüber hinaus lässt sich mit einer solchen Prismenmatrix erreichen, dass ein Proband auch bei einem Verändern der Kopfhaltung oder des Abstands zu dem Sehprüfgerät in dem linken und rechten Auge voneinander exakt getrennte Teilbilder zur Anzeige gebracht werden können.

Das Sehprüfgerät kann z.B. in einem Untersuchungssystem eingesetzt werden, das einen Probandenbereich hat, in dem ein Proband für das Durchführen einer Sehprüfung in einem Abstand A von dem Display des Sehprüfgeräts positioniert werden kann, für den gilt: $1m \leq A \leq 7m$, vorzugsweise $2m \leq A \leq 3m$. Bevorzugt gibt es in der Untersuchungseinrichtung eine in dem Probandenbereich angeordnete Sitzeinrichtung.

Die Optikbaugruppe kann allerdings für das Trennen des Lichts der ersten und zweiten Gruppe ausgewählter Zonen des Displays in einer alternativen Ausführungsform der Erfindung auch ein als Parallaxebarriere wirkendes Blendensystem enthalten. Hierfür kann das Blendensystem z.B. als eine Maske mit abwechselnd lichtdurchlässigen und lichtundurchlässigen Bereichen ausgebildet sein.

Für das Festlegen des Verlaufs des optischen Strahlengangs zu den Augen des Probanden ist es von Vorteil, wenn die Optikbaugruppe einstellbar ist. Damit wird ein Anpassen des mit dem Sehprüfgerät bei dem Probanden hervorrufbaren Seheindrucks an die Position des Probanden ermöglicht.

Die horizontale Breite $B_{Mn}$ der lichtundurchlässigen Bereiche der Maske in dem Blendensystem ist bevorzugt wenigstens doppelt so groß ist wie die Breite $B_{Md}$ der lichtdurchlässigen Bereiche der Maske. Mit dieser Maßnahme wird erreicht, dass die dem linken und dem rechten Auge des Probanden mit dem Display angezeigte Information für den Proband nicht nur bei einer nach vorne oder hinten geneigten Kopfstellung für das linke und rechte Auge exakt getrennt ist, sondern auch wenn der Kopf eines Probanden zur Seite gekippt ist. Das erfindungsgemäße Sehprüfsystem ist damit im Hinblick auf das Trennen der Bildinformation für das linke und rechte Auge eines Probanden für dessen Kopfhaltung weit weniger empfindlich als herkömmliche Sehprüfgeräte, die auf dem Prinzip der Trennung dieser Bildinformation mittels polarisiertem Licht beruhen.

Indem das Blendensystem relativ zu dem Display senkrecht und/oder parallel bewegbar ist, insbesondere indem der Abstand z zwischen der das Licht für das linke und rechte Auge trennenden Lichtdurchtrittsebene des Blendensystems und dem Display variiert werden kann, lässt sich das Sehprüfgerät für unterschiedliche Abstände und Kopfhaltungen des Probanden einstellen.

Von Vorteil ist es insbesondere, wenn das Sehprüfgerät eine Einrichtung für das Erfassen der Winkelposition der Augen eines Probanden im Bezug auf die Maske enthält und mit einem Antrieb verbunden ist, mit dem das Blendensystem aufgrund einer erfassten Winkelposition der Augen des Probanden bevorzugt parallel zu der Ebene des Displays, alternativ oder zusätzlich aber auch senkrecht zu der Ebene des Displays, so verlagert werden kann, dass die Ablage S des Zentrums der senkrechten Projektion der Pupillendistanz der Augen des Probanden von einer vertikalen Linie durch die geometrische Mitte der Maske vor dem Verlagern und eine vertikale Verschiebung V der geometrischen Mitte der Maske nach dem Verlagern der folgenden Beziehung genügt:

$$\frac{V}{z} = \frac{S}{g}.$$

Dabei ist z der Abstand der Lichtdurchtrittsebene des Blendensystems von dem Display und g der Abstand des Probanden von der Lichtdurchtrittsebene des Blendensystems. Ein solches Sehprüfgerät kann insbesondere für das automatische nachführen der Maske ausgelegt sein, um einem Probanden Testmuster zur Anzeige zu bringen, die für das linke und rechte Auge getrennt sind, auch wenn der Proband seine Kopfhaltung ändert.

Für das Bewegen des Blendensystems relativ zu dem Display ist z.B. ein motorischer, insbesondere ein elektromotorischer, ein piezoelektrischer, ein magnetostriktiver oder auch ein mikromechanischer Antrieb geeignet.

Günstig ist es, wenn das Display eine Vielzahl von Pixel für das Erzeugen von Bildpunkten in aneinander liegenden, zueinander komplementären streifenförmigen Displayzonen aufweist, die vorzugsweise in vertikaler Richtung erstreckt sind. Unter zueinander komplementären Displayzonen werden dabei Displayzonen verstanden, die das gleichzeitige Erzeugen von unterschiedlichen Teilbildern auf einem Display ermöglichen.

Die abwechselnd lichtdurchlässigen und lichtundurchlässigen Bereiche der Maske können eine Streifenform haben und parallel zu den Displayzonen angeordnet sein.

Es ist von Vorteil, wenn die Breite $B_{Mu}$ der für das Licht des Displays undurchlässigen Bereiche der Maske und die Breite $B_{Md}$ der für das Licht des Displays durchlässigen Bereiche der Maske und die Breite $B_D$ der streifenförmigen Displayzonen folgender Beziehung genügen:

$$B_{Mu} \approx 3\, B_{Md} \approx 3/2\, B_D,$$

wobei der Abstand z der Maske von dem Display dem 25- bis 50-fachen der Breite $B_D$ der Displayzonen entspricht.

Von Vorteil ist es insbesondere, wenn die Breite $B_D$ der streifenförmigen Displayzonen und/oder die Breite $B_M$ der Streifen der Streifenmaske eingestellt werden kann. Damit ist es möglich, das Sehprüfgerät an die Pupillendistanz der Augen eines Probanden und die Kopfposition des Probanden anzupassen. Die Breite $B_D$ der streifenförmigen Displayzonen entspricht bevorzugt dem Durchmesser P eines Displaypixels für das Erzeugen eines Bildpunkts.

Eine Idee der Erfindung ist es auch, das Sehprüfgerät und / oder die Untersuchungseinrichtung für das Durchführen des Kreuztests oder des Zeigertests oder des Rechtecktests oder des Dreieckstests oder des Stereo-Sehgleichgewichtstests einzusetzen. Darüber hinaus erstreckt sich die Erfindung auch auf das Überprüfen von Sehfunktionen der Augen eines Probanden mit einem Sehprüfgerät oder mit einer Untersuchungseinrichtung, bei welchem mit der ersten und der zweiten Gruppe ausgewählter Bereiche des Displays zwei wenigstens abschnittsweise zueinander komplementäre Teilmuster insbesondere für das Durchführen des Kreuztests oder des Rechtecktests oder des Dreieckstests oder des Stereo-Sehgleichgewichtstests erzeugt werden und dabei eines der beiden Teilmuster dem linken Auge und das andere Teilmuster dem rechten Auge des Probanden zur Anzeige gebracht wird.

Im Folgenden wird die Erfindung anhand der in der Zeichnung in schematischer Weise dargestellten Ausführungsbeispiele näher erläutert.

Es zeigen:

Fig. 1      eine erste Untersuchungseinrichtung mit einem Sehprüfgerät;

Fig. 2      das Erfassen der Winkelposition der Augen eines Probanden mit einer Kamera in dem Sehprüfgerät;

Fig. 3      eine Draufsicht auf die optisch aktive Seite eines Displays in dem Sehprüfgerät;

Fig. 4      eine Draufsicht auf ein Blendensystem in dem Sehprüfgerät;

Fig. 5      einen Schnitt des Sehprüfgeräts entlang der Linie IV - IV in Fig. 1;

Fig. 6a      eine Anzeige eines Teilmusters an dem Display für ein rechtes Probandenauge;

Fig. 6b      eine Anzeige eines Teilmusters an dem Display für ein linkes Probandenauge;

Fig. 7a   bis 7d verschiedene Testmuster die in der Untersuchungseinrichtung einem Probanden zur Anzeige gebracht werden können;

Fig. 8   eine alternative Ausführung einer Maske in einem Blendensystem des Sehprüfgeräts;

Fig. 9   eine weitere alternative Ausführung einer Maske in einem Blendensystem des Sehprüfgeräts;

Fig. 10   eine weitere Untersuchungseinrichtung mit einem Sehprüfgerät, das ein Display mit einer Prismenmatrix enthält; und

Fig. 11   einen Schnitt des Sehprüfgeräts mit der Prismenmatrix entlang der Linie VIII-VIII in Fig. 10.

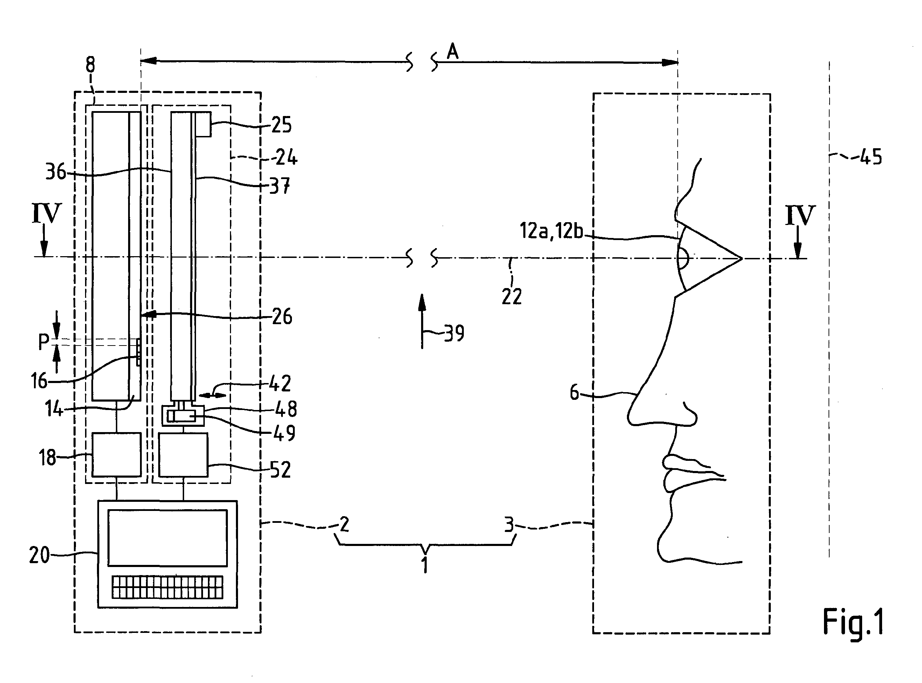

Die in Fig. 1 gezeigte Untersuchungseinrichtung 1 umfasst ein Sehprüfgerät 2 und hat einen Probandenbereich 3. Das Sehprüfgerät 2 ist dazu ausgelegt, den Augen 4 eines Probanden 6 in dem Probandenbereich 3 Testmuster anzuzeigen, die mit einer Bilderzeugungsvorrichtung 8 generiert werden. In dem Probandenbereich 3 der Untersuchungseinrichtung 1 befindet sich eine Sitzeinrichtung (nicht dargestellt). Diese Sitzeinrichtung ermöglicht dem Probanden 6 ein entspanntes Betrachten eines mit der Bilderzeugungsvorrichtung 8 erzeugten Testmusters. Der Probandenbereich 3 ist von dem Sehprüfgerät 2 derart beabstandet angeordnet, dass die Augen 12a, 12b des Probanden 6 in dem Probandenbereich 3 z.B. den Abstand A ≈ 2,5 m von der Bilderzeugungsvorrichtung 8 haben. In einer solchen Untersuchungseinrichtung kann aber grundsätzlich auch ein Abstand A der Augen des Probanden von der Bilderzeugungsvorrichtung 8 vorgesehen sein, der in dem Bereich 1m ≤ A ≤ 7m oder auch darüber liegt.

Die Bilderzeugungsvorrichtung 8 enthält ein Display 14. Das Display 14 ist als LED-Matrix ausgebildet. Für das Erzeugen von Testmustern hat das Display 14 eine Vielzahl von Pixel 16, die über eine Treiberbaugruppe 18 mit einer Rechnereinheit 20 angesteuert werden können. Das bei entsprechendem Ansteuern des Displays 14 von den Pixeln 16 ausgesendete Licht wird den Augen 12a, 12b des Probanden 6 mit einem optischen Strahlengang 22 zugeführt. In einer alternativen Ausführungsform des Untersuchungssystems 1 ist es auch möglich, dem Probanden 6 die Anzeige des Displays 14 mit einem optischen Strahlengang zuzuführen, der über einen oder mehrere Spiegel geleitet ist.

Mit dem Sehprüfgerät 2 kann den Augen 12a, 12b des Probanden 6 ein Testmuster visualisiert werden, das aus einem dem linken Auge 12a zugeführten Teilmuster und einem dem rechten Auge 12b zugeführten Teilmuster zusammengesetzt ist. Das Teilmuster für das linke Auge 12a kann dabei mit dem Sehprüfgerät 2 dem Proband 6 unabhängig von dem Teilmuster für das rechte Auge 12b zur Anzeige gebracht werden.

Für das unabhängige Visualisieren von Testmustern gibt es in dem Sehprüfgerät 2 eine Optikbaugruppe 24. Die Optikbaugruppe 24 enthält ein Blendensystem 36, um das Licht von dem Display 14 für das linke und rechte Auge 12a, 12b des Probanden aufzutrennen. Das Blendensystem 36 umfasst eine Maske 37, die sich in der mit dem Pfeil 39 angedeuteten vertikalen Richtung erstreckende streifenförmige Bereiche hat, die für das Licht des Displays abwechselnd durchlässig und undurchlässig sind. Die Optikbaugruppe 24 trennt das Licht, welches von einer ersten Gruppe ausgewählter Bereiche des Displays 14 dem optischen Strahlengang 22 zugeführt wird, von dem Licht, das der Strahlengang 22 von einer zweiten Gruppe ausgewählter Bereiche des Displays 14 erhält. In dem Sehprüfgerät 2 gibt es eine Kamera 25. Die Kamera 25 ist mit dem Computer 20 verbunden.

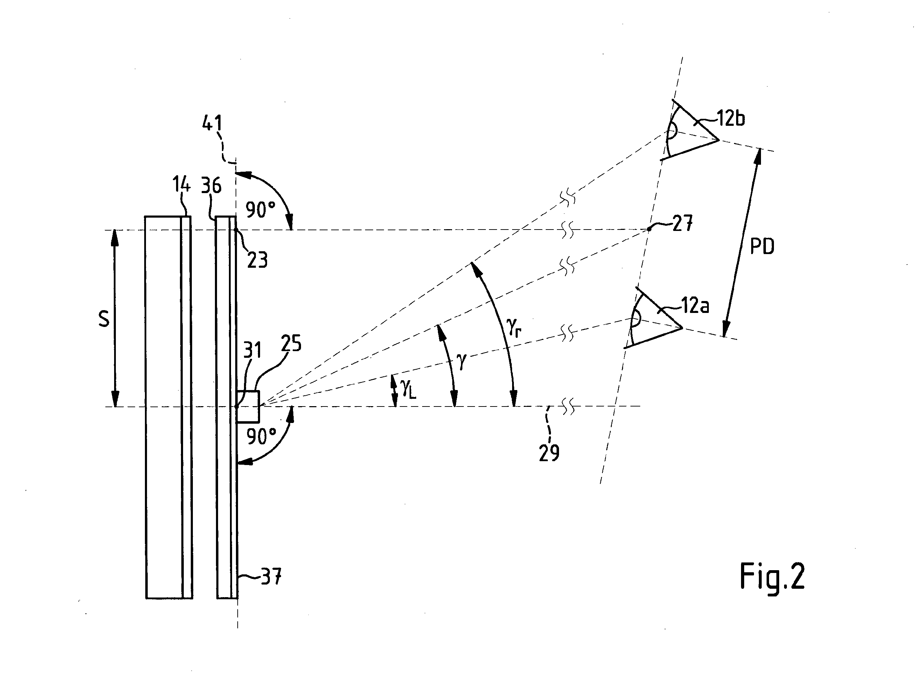

Die Fig. 2 zeigt einen Abschnitt des Sehprüfgeräts 2 in einer Ansicht von oben. Mit der Kamera 25 kann die Winkelposition der Augen 12a, 12b des Probanden 6 im Bezug auf die Maske 37 erfasst werden. Der Computer 20 in dem Sehprüfgerät 2 enthält hierzu ein Bildauswerteprogramm. Damit kann aufgrund eines mit der Kamera 25 erfassten Bildes des Probanden der Winkel

$$\gamma := \frac{1}{2}\{\gamma_l + \gamma_r\}$$

des Zentrums 27 der Pupillendistanz PD des Probanden zu der Flächennormalen 29 auf der vertikalen Linie 31 durch das geometrische Zentrum, d.h, die Mitte der Maske 37 bestimmt werden. Der Computer 20 ermittelt hierfür mit dem Programm zunächst das Zentrum der Pupillen 31 a, 31 b der Augen 12a, 12b des Probanden. Er bestimmt daraus dann den Winkel γ, unter dem die Kamera 25 das Zentrum 27 der Pupillendistanz PD im Bezug auf die Flächennormale 29 durch die geometrische Mitte der Maske 37 erfasst.

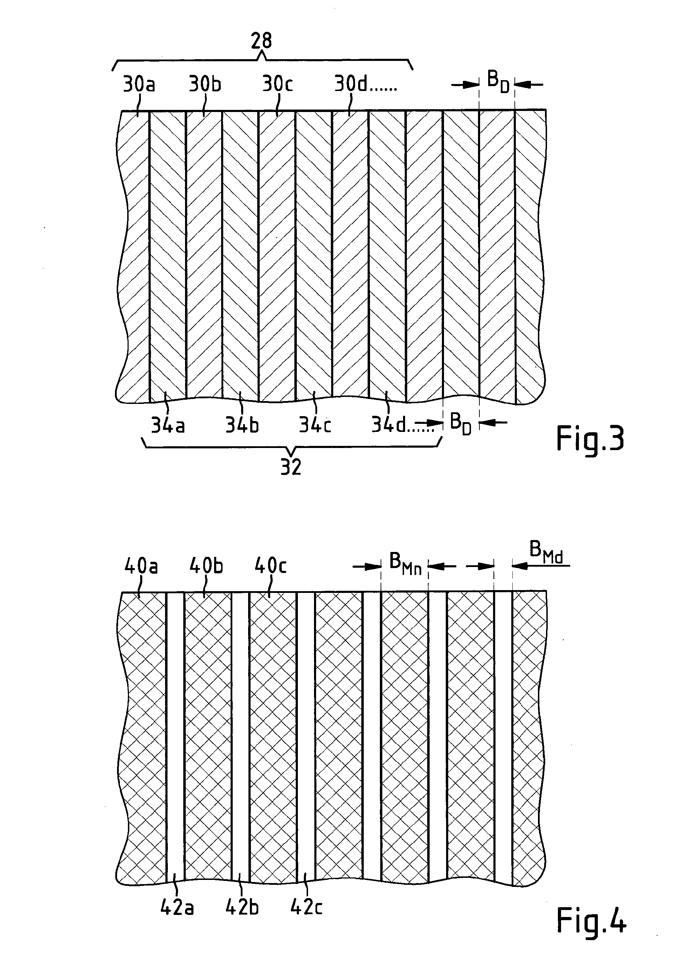

Die Fig. 3 zeigt einen Abschnitt der optisch aktiven Seite 26 des Displays 14 in dem Sehprüfgerät 2. Das Display 14 hat eine erste Gruppe 28 von streifenförmigen Display-Zonen 30a, 30b, 30c, 30d .... und weist eine zweite Gruppe 32 von streifenförmigen Display-Zonen 34a, 34b, 34c, 34d ... auf.

Die Display-Zonen 34a, 34b, 34c, 34d, ... sind zu den Display-Zonen 30a, 30b, 30c, 30d, ... komplementär. D.h., die Fläche wenigstens einer der Display-Zonen 34a, 34b, 34c, 34d und die Fläche wenigstens einer der Display-Zonen 34a, 34b, 34c, 34d, ... berühren einander und überdecken das Display 14 in wenigstens einem Abschnitt zusammenhängend.

Die Display-Zonen 30, 34 können mit der Rechnereinheit 20 des in der Fig. 1 gezeigten Sehprüfgeräts ausgewählt werden. Mit der Rechnereinheit 20 wird in den Display-Zonen 30a, 30b, 30c, ... ein Teilmuster für das linke Auge 12a des Probanden 6 erzeugt. Entsprechend wird mit den Display-Zonen 32 ein Teilmuster für das rechte Auge 12b des Probanden generiert. Das in den Display-Zonen 28 erzeugte Teilmuster ist dabei zu dem Teilmuster, das in den Display-Zonen 32 zur Anzeige gebracht wird, komplementär. Die Display-Zonen 30a, 30b ... und 34a, 34b, 34c haben eine konstante Breite $B_D$. Die Breite $B_D$ entspricht dem Durchmesser P eines Pixels 16 auf dem Display 14, d.h. dem minimalen Durchmesser eines Bildpunkts in einem Bild, das mit dem Display 14 angezeigt werden kann.

Die Fig. 4 zeigt einen Abschnitt der Maske 37 in einer Draufsicht. Die Maske 37 ist eine Streifenmaske. Die Maske 37 wirkt als Parallaxebarriere. Die streifenförmigen Bereiche 40a, 40b, 40c, ..... der Maske 37 sind für das Licht des Displays 14 undurchlässig. Umgekehrt sind die Bereiche 42a, 42b, 42c, ..... der Maske 37 des Blendensystems 36 für das Licht aus dem Display 14 durchlässig. Die Breite $B_{Md}$ der für das Licht des Displays 14 durchlässigen Bereiche 42a, 42b, 42c, ... beträgt $B_{Md} = \frac{1}{2} B_D$. Die Breite $B_{Mn}$ der für das Licht undurchlässigen Bereiche 40a, 40b, 40c, .... der Maske 37 ist eineinhalbmal so groß wie die Breite $B_D$ der Display-Zonen 30a, 30b, 30c, ..., d.h. $B_{Mn} = \frac{3}{2} B_D$. Die Maske 37 hat damit eine Translationsinvarianz. Durch Verlagern in horizontaler Richtung um den Weg

$$S := B_{Md} + B_{Mn}$$

wird die Struktur der Maske 37 in sich selbst überführt.

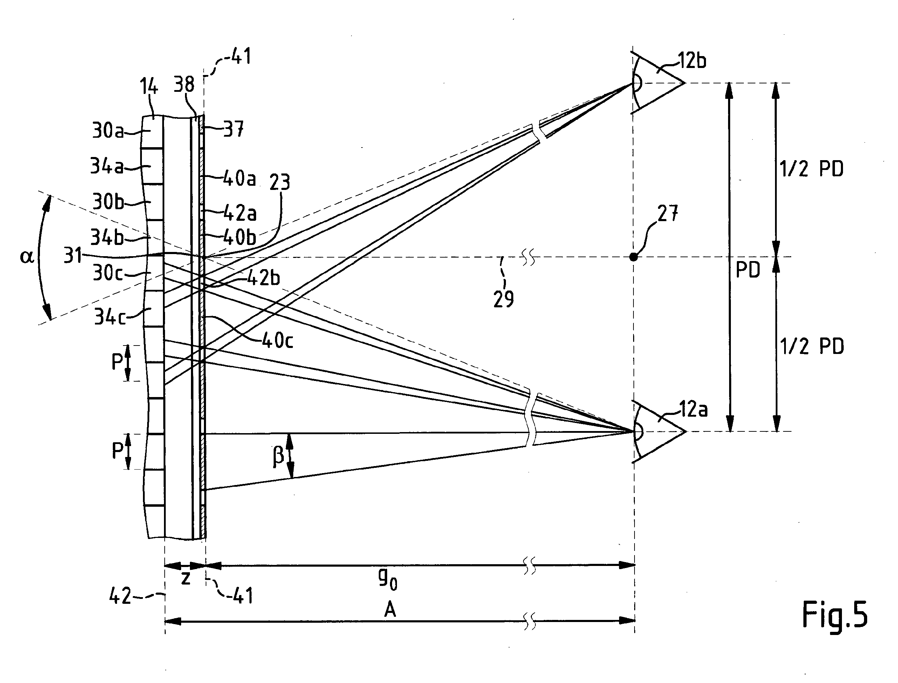

Die Fig. 5 ist ein Teilschnitt des Sehprüfgeräts entlang der Linie IV-IV in Fig. 1. Die Maske 37 des Blendensystems 36 ist auf einem durchsichtigen Trägerelement 38 angeordnet. Mit den für das Licht des Displays 14 durchlässigen und undurchlässigen streifenförmigen Bereichen 40a, 40b, 40c; 42a, 42b, 42c trennt die Maske 37 das Licht für das linke und rechte Auge 12a, 12b des Probanden 6 in der Lichtdurchtrittsebene 41.

Die Lichtdurchtrittsebene 41 der Maske 37 ist in dem Sehprüfgerät 2 frei verlagerbar. Hierzu enthält das Sehprüfgerät 2, wie in der Fig. 1 gezeigt, eine Verstelleinrichtung 48 für das Blendensystem 36. Mit der Verstelleinrichtung 48 kann das Blendensystem 36 entsprechend dem in der Fig. 1 gezeigten Doppelpfeil 42 in der horizontalen Richtung und in der zu der Richtung des Doppelpfeils 42 senkrechten horizontalen Richtung bewegt werden. Für das Verstellen enthält die Verstelleinrichtung 48 einen piezoelektrischen Antrieb 49, der über eine Treiberbaugruppe 52 mittels des Rechners 20 in Abhängigkeit der mit der Kamera 25 erfassten Winkelposition der Augen 12a, 12b des Probanden 6 von dem Display 14 gesteuert werden kann. Die Verstelleinrichtung 48 ermöglicht es, den Abstand z der Ebene 42 des Displays 14 von der Lichtdurchtrittsebene 41 der Blende 37 in dem Bereich 8mm $\leq$ z $\leq$ 15mm zu variieren. Entsprechend der mittels dem Computer 20 und der Kamera 25 erfassten Zentrum 27 der Pupillendistanz PD wird mit der Verstelleinrichtung 48 das Blendensystem 36 so eingestellt und nachgeführt, dass die Gerade 29 durch die Punkte 27 und die vertikale Linie 31 hinter der Blende 37 auf der Ebene des Displays 14 die Grenze zweier benachbarter Display-Zonen trifft. Die Position der Display-Zonen 30a, 30b, 30c, ... und der Display-Zonen 34a, 34b, 34c, ... des Displays 14 wird dabei der Verlagerung der Maske 37 angepasst. Für das Verstellen des Blendensystems 36 bestimmt der Computer 20 aus dem Winkel $\gamma$, unter dem die Kamera 25 das Zentrum 27 der Pupillendistanz PD in Bezug auf die Flächennormale 29 in dem Zentrum 31 der Maske 37 erfasst, eine günstige horizontale Verschiebung V der Maske 37 parallel zu dem Pfeil 39, d.h. senkrecht zu der Längsrichtung der in der Fig. 4 gezeigten streifenförmigen Bereiche der Maske 37 und parallel zu der Lichtdurchtrittsebene 41 mit

$$V := z \ \tan \gamma \ .$$

Die günstige Verschiebung der Maske 37 genügt also folgender Beziehung:

$$\frac{V}{z} = \frac{S}{g}$$

Dabei ist S die in der Fig. 2 gezeigte Ablage des Zentrums 23 der senkrechten Projektion der Pupillendistanz PD in der der Ebene 41 der Maske 37 von der Flächennormale 29 auf der vertikalen Linie 29. g ist der Abstand des Probanden von der Lichtdurchtrittsebene 41 des Blendensystems 36.

In einer zu der vorstehend beschriebenen Ausführungsform modifizierten Ausführungsform der Erfindung kann das Blendensystem 36 auch zusätzlich in der zu dem Doppelpfeil 42 senkrechten vertikalen Richtung verlagert werden.

Der Proband 6 mit der Pupillendistanz PD sieht die Display-Zonen 30a, 30b, 30c, ... und 34a, 34b, 34c, ... mit dem linken Auge 12a und dem rechten Auge 12b voneinander getrennt, wenn der folgenden geometrischen Beziehung genügt wird:

$$\tan\left(\alpha/2\right) = \frac{PD}{2g_0} \ = \frac{P}{2z} \ ,$$

wobei $\alpha$ der Sehwinkel ist, unter dem der Proband 6 mit dem linken und rechten Auge 12a, 12b die Maske 37 des Blendensystems 36 unter dem Abstand $g_0$ erfasst, und

P die Breite einer streifenförmigen Display-Zone 30a, 30b, 30c, ...; 34a, 34b, 34c .. des Displays 14 ist.

Die Erfinder haben herausgefunden, dass unter Annahme infinitesimal schmaler, für das Licht des Displays 14 durchlässiger Bereiche 42a, 42b, 42c, ... ein Proband 6 die Display-Zonen 30a, 30b, 30c, ... und 34a, 34b, 34c, ... in dem Abstandsbereich $\Delta$ mit

$$\frac{1}{2} g_0 \leq g_0 \mp \Delta \leq \frac{3}{2} g_0$$

exakt voneinander getrennt wahrnehmen kann.

Die Erfinder haben auch erkannt, dass insbesondere unter dem Abstand $g_0$ von der Maske 37 ein Proband 6 die Display-Zonen 30a, 30b, 30c, ... und 34a, 34b, 34c, ... auch exakt voneinander getrennt wahrnehmen kann, wenn der Proband 6 seinen Kopf um die in der Fig. 1 gezeigte vertikale Achse 45 um einen Winkel $\varphi$ von bis zu $\varphi = \pm 60°$ zur linken oder zur rechten Seite dreht. Die Erfinder haben weiter erkannt, dass der Proband 6 dann die Display-Zonen 30a, 30b, 30c, ... und 34a, 34b, 34c, ... darüber hinaus voneinander exakt getrennt wahrnehmen kann, wenn er seinen Kopf gegenüber der in der Fig. 1 gezeigten Achse 45 um einen Winkel $\theta$ von bis zu $\theta = \pm 60°$ zur Seite neigt.

Die Erfinder haben außerdem erkannt, dass bei einer endlichen Breite $B_{Md}$ der für das Licht des Displays 14 durchlässigen Bereiche die Ausdehnung diese Abstandsbereichs um den Faktor $k = 1 - \frac{B_{Md}}{B_{Mu}}$ verringert wird, wobei $B_{Mu}$ die Breite der für das Licht undurchlässigen Bereiche der Maske 37 ist. D.h., für den Abstandsbereich $\Delta'$, in dem der Proband 6 ein in den vorgenannten Display-Zonen erzeugtes Bild getrennt wahrnimmt, gilt:

$$\Delta` = \left(1 - \frac{B_{Md}}{B_{Mu}}\right) \Delta.$$

In der in Fig. 1 gezeigten Untersuchungseinrichtung 1 ist die Breite $B_{Mu}$ und $B_{Md}$ der für das Licht undurchlässigen bzw. durchlässigen Bereiche 40a, 40b, 40c, ..; 42a, 42b, 42c, ... der Maske 37 in dem Sehprüfgerät 2 und die Entfernung des Probandenbereichs 6 von dem Sehprüfgerät 2 so gestaltet, dass der Sehwinkel $\beta$, unter dem ein Proband 6 in dem Abstand A von dem Display 14 mit $1\,m \leq A \leq 5m$ einen Bereich 40a erfasst, kleiner ist als eine Bogenminute, d.h. $\beta \leq 1'$. Diese Maßnahme gewährleistet, dass ein typisches Probandenauge die Streifenstruktur der Maske 37 nicht mehr auflösen kann.

Für einen Durchmesser P = 0,3 mm eines Pixels 16 des Displays 14 und für die Breite $B_{Md} = \frac{1}{2}$ P und $B_{Mu} = \frac{3}{2}$ P der stufenförmigen, für das Licht durchlässigen bzw. undurchlässigen Bereiche der Maske 37 und einer Pupillendistanz PD der Augen 12a, 12b des Probanden 6 in dem Bereich 60mm $\leq$ PD $\leq$ 70 mm können also für einen Abstandsbereich $2m \leq g_0 \mp \Delta \leq 3m$ der Augen 12a, 12b des Probanden 6 von der Lichtdurchtrittsebene 41 der Maske 37 die mittels des Displays 14 in den Display-Zonen 30a, 30b, 30c, ...; 34a, 34b, 34c, ... erzeugten Teilmuster voneinander getrennt zur Anzeige gebracht werden. Von dem Probanden 6 werden die in den Displayzonen 30a 30b, 30c, ... und 34a, 34b, 34c, ... erzeugten Teilmuster dann als zueinander komplementäre Teilmuster wahrgenommen.

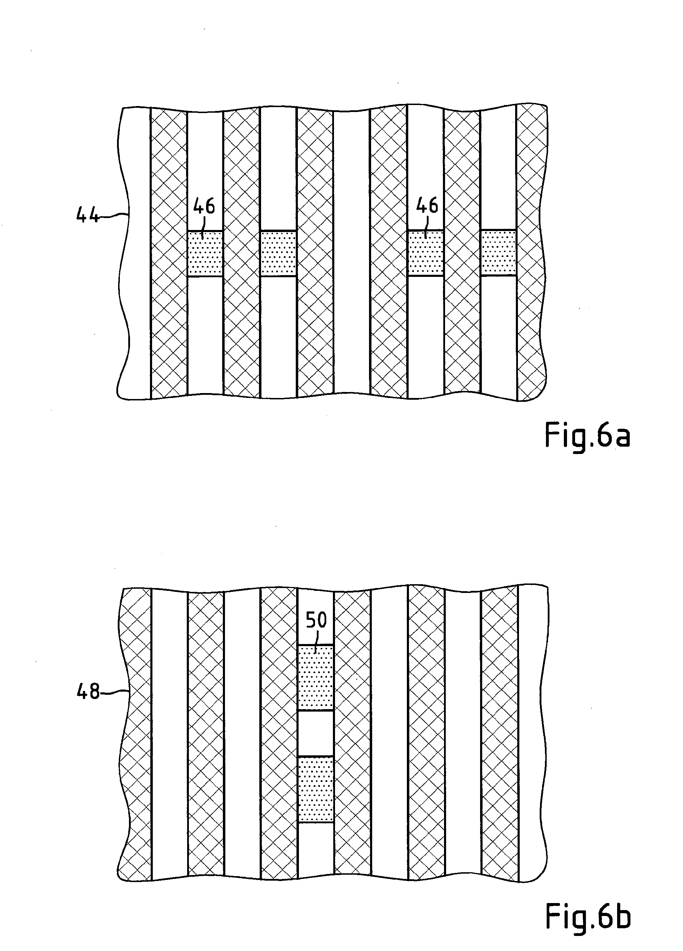

Die Fig. 6a zeigt einen Abschnitt 44 des Displays 14 mit einem für das rechte Auge 12b des Probanden 6 erzeugten balkenförmigen Teilmuster 46. In der Fig. 6b ist mittels des Displays 14 ein balkenförmiges Teilmuster 50 zur Anzeige für das rechte Auge 12a des Probanden 6 dargestellt.

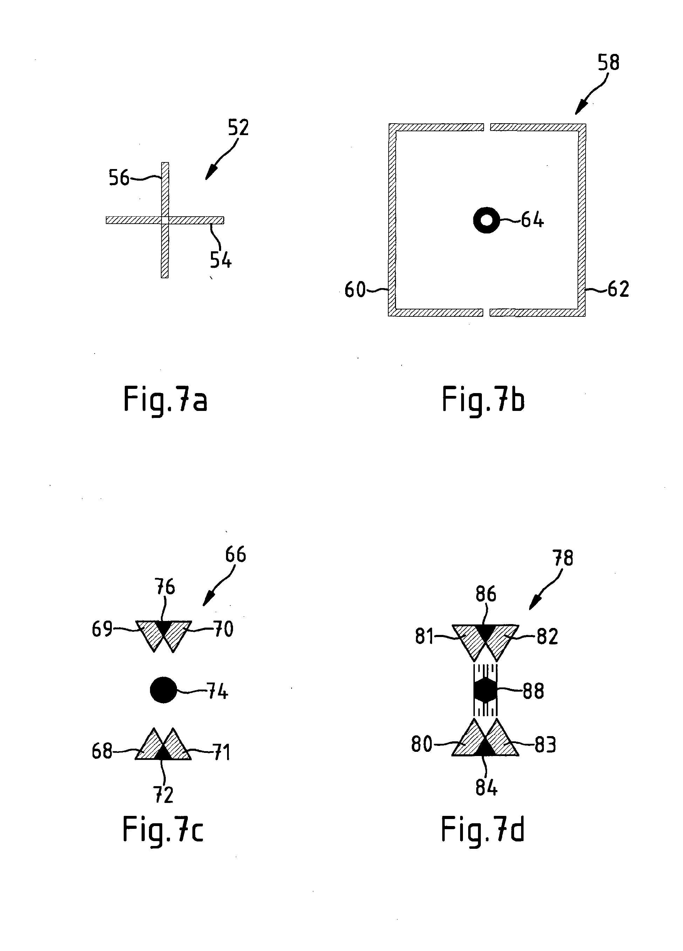

Die Fig. 7a zeigt das bei einer Anzeige der Teilmuster 48, 50 mittels des Displays 14 von einem Probanden wahrgenommene Testmuster 52. Mit dem Testmuster 52 kann die Winkelfehlsichtigkeit eines Probanden mit dem z.B. auf S. 248 in dem Handbuch für Augenoptik, herausgegeben von Carl Zeiss, 4. Auflage 2000, beschriebenen Kreuztest untersucht werden. Bei dem Kreuzest erfasst der Proband den Abschnitt 54 des Musters mit einem Auge, z.B. dem linken, und den Abschnitt 56 hiervon getrennt mit dem anderen.

Die Fig. 7b zeigt ein Testmuster 58. Mit dem Testmuster 58 kann die Winkelfehlsichtigkeit eines Probanden mit dem Rechtecktest untersucht werden, der ebenfalls z.B. auf S. 248 in dem Handbuch für Augenoptik, herausgegeben von Carl Zeiss, 4. Auflage 2000, beschrieben ist. Bei dem Rechteck-Test erfasst der Proband den Abschnitt 60 des Musters mit einem Auge, den Abschnitt 62 mit dem anderen, und den Abschnitt 64 mit beiden Augen. Um das Testmuster 58 mit dem Sehprüfgerät 2 zu visualisieren, wird z.B. der Abschnitt 60 und der Abschnitt 64 an dem Display 14 in den Display-Zonen 30a, 30b, 30c, ... zur Anzeige gebracht. Der Abschnitt 62 sowie der Abschnitt 64 wird in den Display-Zonen 34a, 34b, 34c, ... angezeigt.

Die Fig. 7c zeigt ein weiteres Testmuster 66. Mit dem Testmuster 66 kann die Winkelfehlsichtigkeit eines Probanden mit dem Dreieckstest überprüft werden, der ebenfalls z.B. auf S. 248 in dem Handbuch für Augenoptik, herausgegeben von Carl Zeiss, 4. Auflage 2000, beschrieben ist. Bei dem Dreieckstest erfasst der Proband die Abschnitte 68, 69 des Musters mit einem Auge, die Abschnitte 70, 71 mit dem anderen, und die Abschnitte 72, 74, 76 mit beiden Augen. Um das Testmuster 66 mit dem Sehprüfgerät 2 zu visualisieren, werden z.B. die Abschnitte 68 und 72, 74, 76 an dem Display 14 in den Display-Zonen 30a, 30b, 30c, .. zur Anzeige gebracht und die Abschnitte 70, 72, 74, 76 in den Display-Zonen 34a, 34b, 34c, ....

Das in der Fig. 7d gezeigte Testmuster 78 dient für das Untersuchen der Winkelfehlsichtigkeit eines Probanden mit dem Stereo-Gleichgewichtstest, der z.B. auch auf S. 248 in dem Handbuch für Augenoptik, herausgegeben von Carl Zeiss, 4. Auflage 2000, erläutert ist. Bei dem Stereo-Gleichgewichtstest erfasst der Proband die Abschnitte 80, 81 des Musters mit einem Auge, die Abschnitte 82, 83 mit dem anderen und die Abschnitte 84, 86 und 88 mit beiden Augen. Um das Testmuster 78 mit dem Sehprüfgerät 2 zu visualisieren, werden z.B. die Abschnitte 80, 81 und 84, 86, 88 an dem Display 14 in den Display-Zonen 30a, 30b, 30c, ... zur Anzeige gebracht und die Abschnitte 82, 83 sowie 84, 86, 88 in den Display-Zonen 34a, 34b, 34c, ....

Es sei bemerkt, dass anders als in der Darstellung der Fig. 6a bis 6d die verschiedenen Abschnitte der dort gezeigten Testmuster 52, 58, 66 und 78 von einem Proband je nach Art der Sehstörung mit horizontalem und/oder vertikalem Versatz, gegeneinander verdreht oder auch unterschiedlich groß wahrgenommen werden können.

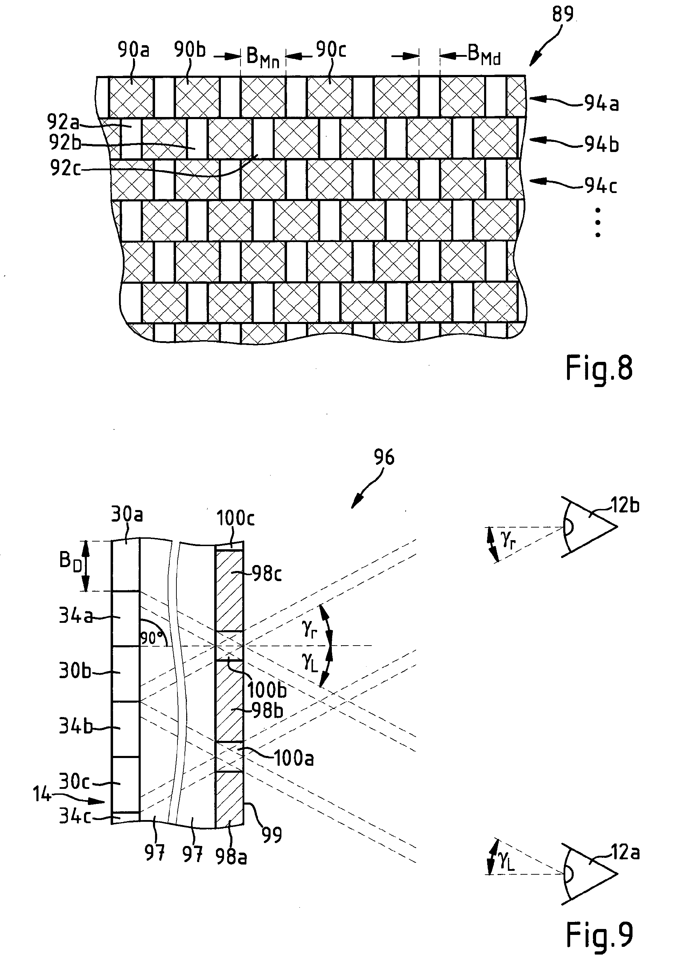

Die Fig. 8 zeigt einen Abschnitt einer alternativen Ausführung einer Maske 89 für das Blendensystem 36. Die Maske 89 hat zueinander versetzt angeordnete Bereiche 90a, 90b, 90c, ...., die für das Licht des Displays 14 undurchlässig sind. Die Bereiche 92a, 92b, 92c, ... der Maske 89 sind komplementär zu den Bereichen 90a, 90b, 90c, .... Für das Licht des Displays 14 sind die Bereiche 92a, 92b, 92c, ... durchlässig.

Die Bereiche 90a, 90b, 90c, ... einerseits und die Bereiche 92a, 92b, 92c, ..., 94b, 94c, ... andererseits haben jeweils eine Rechteckform. Die Bereiche 90a, 90b, 90c, ... und 92a, 92b, 92c sind in aufeinanderfolgenden Zeilen 94 angeordnet. In zueinander benachbarten Zeilen 94a, 94b; 94b, 94c sind die für das Licht durchlässigen Bereiche 92a, 92b, 92c, .. zu den für das Licht undurchlässigen Bereichen 90a, 90b, 90c, ... versetzt positioniert. Die Breite $B_{Mu}$ der für das Licht undurchlässigen Bereiche 92a. 92b. 92c, ... ist größer als die Breite $B_{Md}$ der Bereiche, die für das Licht durchlässig sind. Bevorzugt gilt hier:

$$B_{Md} = \frac{1}{2}\, B_{Mu}.$$

Die Fig. 9 zeigt eine Maske 96 als alternative Ausführung einer Maske 37 für das Blendensystem 36. Die Maske 96 ist als LCD-Blende 99 gestaltet. Die LCD-Blende hat spaltförmige Bereiche 98a, 98b, 98c, ..., 100a, 100b, 100c, ..., die mittels einer Steuereinrichtung (nicht gezeigt) für das Licht des Displays 14 wahlweise durchlässig oder undurchlässig geschaltet werden können. Die Geometrie, d.h. insbesondere die Breite $B_D$ der Display-Zonen 30a, 30b, 30c, ... ; 34a, 34b, 34c, ... und die Breite $B_{Mn}$, $B_{Md}$ der Bereiche 98a, 98b, 98c, ..., 100a, 100b, 100c, der Maske 96 kann hier mittels des Computers 20 in dem Sehprüfgerät 2 eingestellt werden. Für das Anpassen des Sehprüfgeräts 2 an die Position der Augen eines Betrachters werden hier mittels des in der Fig. 1 gezeigten Computers 20 aufgrund der mittels der Kamera 25 erfassten Winkelposition der Augen 12a, 12b des Probanden 6 die Positionen und Abmessungen der für das Licht durchlässigen Bereiche 98a, 98b, 98c, ..., und der für das Licht undurchlässigen Sektoren 100a, 100b, 100c, .. so eingestellt, dass der Proband 6 mit seinem linken und rechten Auge 12a, 12b mittels des Displays 14 erzeugte Teilmuster sieht, die voneinander exakt getrennt sind.

Die Maske 96 mit der LCD-Blende 99 ist durch einen transparenten Glaskörper 97 mit dem Display 14 verbunden. Die Maske 96 ist in Bezug auf das Display 14 unbeweglich angeordnet. Grundsätzlich kann jedoch auch bei Einsatz der Maske 96 in einem entsprechenden Sehprüfgerät 2 eine Bewegbarkeit im Bezug auf das Display 14 vorgesehen werden.

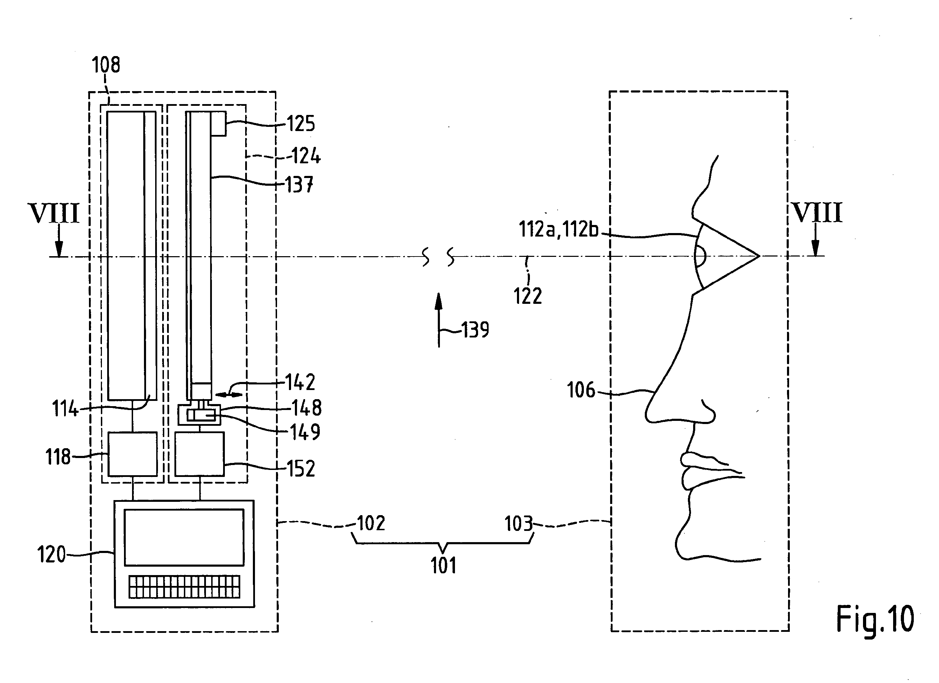

Die Fig. 10 zeigt eine weitere Untersuchungseinrichtung 101 mit einem Sehprüfgerät 102. Auch das Sehprüfgerät 102 ist dazu ausgelegt, den Augen 112a, 112b eines Probanden 106 Testmuster anzuzeigen, die mit einer Bilderzeugungsvorrichtung 108 generiert werden. Soweit die Elemente in der Fig. 10 den Elementen in der Fig. 1 funktional entsprechen, sind diese in der Fig. 10 mit um die Zahl 100 erhöhten Bezugszeichen versehen. Anders als in dem Sehprüfgerät 2 aus Fig. 1 enthält die Optikbaugruppe 124 in dem Sehprüfgerät 102 anstelle des Blendensystems eine Prismenmatrix 137.

Die Prismenmatrix 137 kann gegenüber dem Display 114 verlagerbar sein. Eine solche Verlagerbarkeit ist allerdings nicht zwingend erforderlich. Im Falle einer Verlagerbarkeit der Prismenmatrix 137 ist es günstig, wenn diese insbesondere in einer in Bezug auf die Ebene des Displays 114 senkrechten Richtung entsprechend dem Doppelpfeil 147 und/oder in einer oder mehreren zu dem Doppelpfeil 147 senkrechten Richtungen bewegt werden kann.

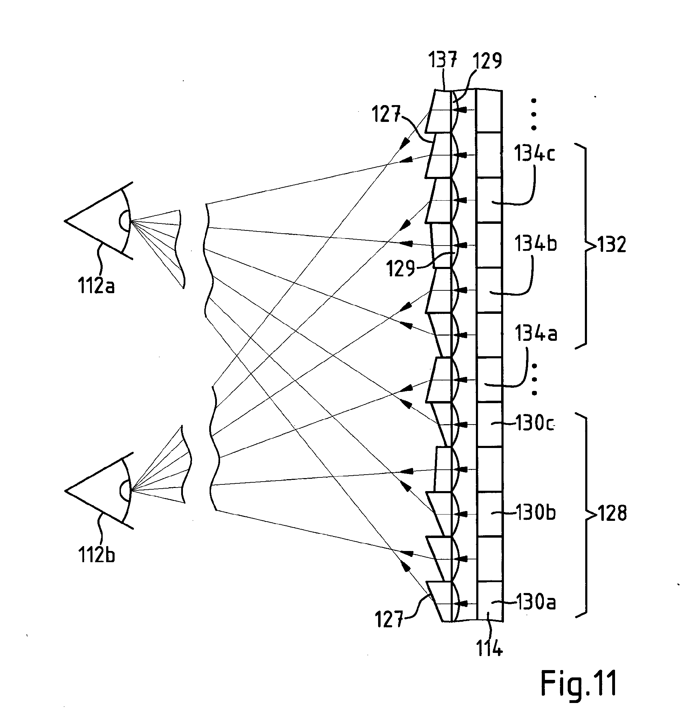

Die Fig. 11 ist ein Teilschnitt des Sehprüfgeräts 102 entlang der Line VIII - VIII in Fig. 10. Die Prismenmatrix 137 hat eine Vielzahl von unterschiedlichen Prismenabschnitten 127, die sich in der vertikalen Richtung 139 erstrecken. Die Prismenabschnitte 127 haben einen dem Display 114 zugewandten Bereich 129, der die Form einer sich in der vertikalen Richtung des Prismenabschnitts 127 erstreckenden konvexen Zylinderlinse hat, die eine zu der vertikalen Richtung 139 parallele Zylinderachse aufweist. Alternativ hierzu ist es auch möglich, anstelle einer Zylinderlinse aufeinanderfolgende Sammellinsen vorzusehen. Mittels der Prismenabschnitte 127 wird das Licht der mit den Pixeln des Displays 114 in der ersten Gruppe 128 der in den Zonen 130a, 130b, 130c erzeugten Bildpunkte aufgrund von Brechung mit einem gerichteten Strahlengang zu dem linken Auge 112a eines Probanden gelenkt, der sich in dem Probandenbereich 103 des Untersuchungssystems 101 befindet. Entsprechend wird das Licht der in der zweiten Gruppe 132 in den Zonen 134a, 134b, 134c, ... erzeugten Bildpunkte aufgrund von Brechung mit einem gerichteten Strahlengang zu dem rechten Auge 112b des Probanden 106 gelenkt. Mittels der zylindrisch geformten Bereiche 129 der Prismenabschnitte oder durch Anordnen eines Linsenarrays an dieser Stelle lässt sich ähnlich wie bei dem anhand der Fig. 1 bis Fig. 9 beschriebenen Sehprüfgerät 2 erreichen, dass für einen Proband 106 in einem bestimmten Abstandsbereich von dem Sehprüfgerät und für unterschiedliche Kopfhaltungen, die in einem gewissen Toleranzbereich liegen, Teilmuster zur Anzeige gebracht werden können, die für das linke und rechte Auge exakt voneinander getrennt sind.

Der Einsatz der Prismenmatrix 137 in dem Sehprüfgerät 102 bietet aufgrund des gerichteten Strahlengangs den Vorteil, dass aus dem Seheindruck zu einem Muster, das einem Probanden in einem Sehtest visualisiert wird, mit großer Zuverlässigkeit auf das Vorliegen oder Nichtvorliegen eines Sehfehlers geschlossen werden kann: Durch eine ungünstige Kopfhaltung bedingte Artefakte in dem Probanden-Seheindruck sind hier nämlich weitgehend ausgeschlossen.

Zusammenfassend sind insbesondere folgende bevorzugte Merkmale der Erfindung festzuhalten: Die Erfindung betrifft ein Sehprüfgerät 2, 102 für das Untersuchen von Winkelfehlsichtigkeiten der Augen 12a, 12b, 112a, 112b eines Probanden 6, 106. Das Sehprüfgerät 2, 102 enthält eine Bilderzeugungsvorrichtung 8, 108, die ein Display 14, 114 für das Erzeugen von Testmustern aufweist, die den Augen 12a, 12b, 112a, 112b des Probanden 6, 106 mit einem optischen Strahlengang 22, 122 zur Anzeige gebracht werden können. In dem Sehprüfgerät ist auf der in dem optischen Strahlengang 22, 122 zu den Augen 12a, 12b, 112a, 112b des Probanden weisenden Seite 26 des Displays 14, 114 eine Optikbaugruppe 24, 124 angeordnet. Die Optikbaugruppe 24, 124 trennt das von einer ersten Gruppe 28, 128 ausgewählter Zonen 30a, 30b, 30c, 30d; 130a, 130b, 130c des Displays 14 dem optischen Strahlengang 22 zugeführte Licht von dem Licht, das dem Strahlengang 22 von einer zweiten Gruppe 32 ausgewählter Zonen 34a, 34b, 34c, 34d; 134a, 134b, 134c des Displays 14 zugeführt ist. Das linke Auge 12a, 112a des Probanden 6, 106 erhält so nur das Licht aus der ersten Gruppe 28, 128 ausgewählter Zonen 30a, 30b, 30c, 30d; 130a, 130b, 130c des Displays 14, 114. Das Licht aus der zweiten Gruppe 32 ausgewählter Zonen 34a, 34b, 34c, 34d; 134a, 134b, 134c des Displays 14, 114 gelangt dann nur zu dem rechten Auge 12b, 112b des Probanden 6, 106. Die Optikbaugruppe 124 umfasst eine Prismenmatrix 125, die eine Vielzahl von in der vertikalen Richtung 139 erstreckte Prismenabschnitte aufweist, die jeweils eine dem Display 114 zugewandten linsenförmigen, insbesondere zylinderlinsenförmigen Bereich mit einer konvexen Oberfläche haben. Die Optikbaugruppe 24 kann alternativ hierzu auch ein spezielles als Parallaxebarriere wirkendes Blendensystem 36 enthalten, mit dem das Licht der ersten und zweiten Gruppe 28, 32; 128, 132 ausgewählter Zonen 30a, 30b, 30c, 30d; 34a, 34b, 34c, 34d des Displays 14, 114 getrennt wird.

**Patentansprüche**

1. Sehprüfgerät (102) für das Untersuchen von Winkelfehlsichtigkeiten der Augen (112a, 112b) eines Probanden (106),

   mit einer Bilderzeugungsvorrichtung (108), die ein Display (114) für das Erzeugen von Testmustern aufweist, welche den Augen (112a, 112b) des Probanden (106) mit einem optischen Strahlengang (122) zur Anzeige gebracht werden können,

   wobei auf der in dem optischen Strahlengang (122) zu den Augen (112a, 112b) des Probanden weisenden Seite des Displays (114) eine Optikbaugruppe (124) angeordnet ist, die das von einer ersten Gruppe (128) ausgewählter Zonen (130a, 130b, 130c) des Displays (114) dem optischen Strahlengang (122) zugeführte Licht von dem Licht trennt, das dem Strahlengang (122) von einer zweiten Gruppe (132) ausgewählter Zonen (134a, 134b, 134c) des Displays (114) zugeführt ist, um dem linken Auge (112a) des Probanden (106) das Licht aus der ersten Gruppe (128) ausgewählter Zonen (130a, 130b, 130c) des Displays (114) zuzuführen und das Licht aus der zweiten Gruppe (132) ausgewählter Zonen (134a, 134b, 134c) des Displays zu dem rechten Auge (112b) des Probanden (106) zu leiten,

   **dadurch gekennzeichnet, dass**

   die Optikbaugruppe (124) eine Prismenmatrix (125) umfasst, die eine Vielzahl von in vertikaler Richtung (139) erstreckten Prismenabschnitten (127) aufweist, die jeweils einen dem Display (114) zugewandten linsenförmigen, insbesondere zylinderlinsenförmigen Bereich (129) mit einer konvexen Oberfläche haben.

2. Sehprüfgerät nach Anspruch 1, **dadurch gekennzeichnet, dass** das Display (114) eine Vielzahl von Pixel (16) für das Erzeugen von Bildpunkten in zueinander komplementären Zonen (130a, 130b, 130c, ...; 134a, 134b, 134c, ...) des Displays (114) aufweist, die in vertikaler Richtung (139) erstreckt sind, und der dem Display (114) zugewandte linsenförmige Bereich (129) der Prismenabschnitte (127) zylinderlinsenförmig ist und eine in die vertikale Richtung (139) verlaufende Zylinderlinsenachse hat.

3. Sehprüfgerät nach Anspruch 1 oder 2, **dadurch gekennzeichnet, dass** die Optikbaugruppe (124) für das Festlegen des Verlaufs des optischen Strahlengangs (122) zu den Augen (112a, 112b) des Probanden (106) einstellbar ist.

4. Sehprüfgerät (2) für das Untersuchen von Winkelfehlsichtigkeiten der Augen (12a, 12b) eines Probanden (6),

mit einer Bilderzeugungsvorrichtung (8), die ein Display (14) für das Erzeugen von Testmustern aufweist, welche den Augen (12a, 12b) des Probanden (6) mit einem optischen Strahlengang (22) zur Anzeige gebracht werden können,

wobei auf der in dem optischen Strahlengang (22) zu den Augen (12a, 12b) des Probanden weisenden Seite (26) des Displays (14) eine Optikbaugruppe (24) angeordnet ist, die das von einer ersten Gruppe (28) ausgewählter Zonen (30a, 30b, 30c, 30d) des Displays (14) dem optischen Strahlengang (22) zugeführte Licht von dem Licht trennt, das dem Strahlengang (22) von einer zweiten Gruppe (32) ausgewählter Zonen (34a, 34b, 34c, 34d) des Displays (14) zugeführt ist, um dem linken Auge (12a) des Probanden (6) das Licht aus der ersten Gruppe (28) ausgewählter Zonen (30a, 30b, 30c, 30d) des Displays (14) zuzuführen und das Licht aus der zweiten Gruppe (32) ausgewählter Zonen (34a, 34b, 34c, 34d) des Displays zu dem rechten Auge (12b) des Probanden (6) zu leiten,

**dadurch gekennzeichnet, dass**

die Optikbaugruppe (24) für das Trennen des Lichts der ersten und zweiten Gruppe (28, 32) ausgewählter Zonen (30a, 30b, 30c, 30d) des Displays (14) ein als Parallaxebarriere wirkendes Blendensystem (36) enthält, das eine Maske (37, 89) mit abwechselnd lichtundurchlässigen und lichtdurchlässigen Bereichen (40a, 40b, 40c, 42a, 42b, 42c; 90a, 90b, 90c, ...; 92a, 92b, 92c; ...) umfasst, und

das Display (14) eine Vielzahl von Pixel (16) für das Erzeugen von Bildpunkten in zueinander komplementären Zonen (30a, 30b, 30c, ...; 34a, 34b, 34c, ...) des Displays (14) aufweist, die in vertikaler Richtung (39) erstreckt sind, wobei

die zueinander komplementären Zonen (30a, 30b, 30c, ...; 34a, 34b, 34c, ...) des Displays (14) streifenförmig sind und die Breite ($B_{Mn}$) der für das Licht des Displays (14) undurchlässigen Bereiche (40a, 40b, 40c, ...) der Maske (37) und die Breite ($B_{Md}$) der für das Licht des Displays (14) durchlässigen Bereiche (42a, 42b, 42c, ...) der Maske (37) sowie die Breite ($B_D$) der streifenförmigen Zonen (30a, 30b, 30c, 34a, 34b, 34c) des Displays (14) folgender Beziehung genügen:

$$B_{Mn} \approx 3\ B_{Md} \approx 3/2\ B_D,$$

wobei der Abstand z der Maske (37) von dem Display (14) dem 25- bis 50-fachen der Breite $B_D$ der Zonen (30a, 30b, 30c, 34a, 34b, 34c) des Displays (14) entspricht.

5. Sehprüfgerät nach Anspruch 4, **dadurch gekenn-** **zeichnet, dass** die Optikbaugruppe (24) für das Festlegen des Verlaufs des optischen Strahlengangs (22) zu den Augen (12a, 12b) des Probanden (6) einstellbar ist.

6. Sehprüfgerät nach einem der Ansprüche 4 oder 5, **dadurch gekennzeichnet, dass** die Breite ($B_D$) der streifenförmigen Zonen (30a, 30b, 30c) des Displays (14) und die Breite ($B_{Md}$, $B_{Mu}$) der für das Licht des Displays (14) durchlässigen und undurchlässigen Bereiche (100a, 100b, ...; 98a, 98b, 98c, ...) der Maske (96) einstellbar ist.

7. Sehprüfgerät nach einem der Ansprüche 4 bis 6, **da-** **durch gekennzeichnet, dass** die Breite ($B_D$) der streifenförmigen Zonen (30a, 30b, 30c, 34a, 34b, 34c) des Displays (14) einem Durchmesser P eines Displaypixels (16) für das Erzeugen eines Bildpunkts entspricht.

8. Sehprüfgerät nach einem der Ansprüche 4 bis 7, **da-** **durch gekennzeichnet, dass** das Blendensystem (36) senkrecht und/oder parallel relativ zu dem Display (14) bewegbar ist, wobei wenigstens der Abstand (z) zwischen einer das Licht für das linke und rechte Auge trennenden Lichtdurchtrittsebene (41) des Blendensystems (36) und dem Display (14) und/ oder eine Lage der abwechselnd lichtdurchlässigen und lichtundurchlässigen Bereiche (40a, 40b, 40c, 42a, 42b, 42c) in einer Lichtdurchtrittsebene (41) des Blendensystems (36) variiert werden kann.

9. Sehprüfgerät nach Anspruch 8, **gekennzeichnet** **durch** eine Einrichtung (25, 20) für das Erfassen der Winkelposition der Augen (12a, 12b) eines Probanden (6), die mit einem Antrieb (49) gekoppelt ist, der das Blendensystem (36) aufgrund einer erfassten Winkelposition der Augen (12a, 12b) des Probanden (6) derart verlagert, dass die Ablage S des Zentrums (23) der senkrechten Projektion der Pupillendistanz (PD) der Augen (12, 12b) des Probanden (6) von einer vertikalen Linie (39) in der Mitte der Maske (37) vor dem Verlagern und eine vertikale Verschiebung V der Mitte der Maske (37) nach dem Verlagern der folgenden Beziehung genügt:

$$\frac{V}{z} = \frac{S}{g_0},$$

wobei z der Abstand der Lichtdurchtrittsebene 41 des Blendensystems 36 von dem Display 14 ist und $g_0$ der Abstand des Probanden von der Lichtdurchtrittsebene 41 des Blendensystems 36.

10. Sehprüfgerät nach einem der Ansprüche 4 bis 9, **da-** **durch gekennzeichnet, dass** das Display (14) eine Vielzahl von Pixel (16) für das Erzeugen von Bildpunkten in zueinander komplementären Zonen (30a, 30b, 30c, ...; 34a, 34b, 34c, ...) des Displays (14) aufweist, die in vertikaler Richtung (39) erstreckt sind.

11. Sehprüfgerät nach Anspruch 10, **dadurch gekennzeichnet, dass** die horizontale Breite ($B_{Mn}$) der lichtundurchlässigen Bereiche (40a, 40b, 40c, ...) der Maske (37) wenigstens doppelt so groß ist wie die Breite ($B_{Md}$) der lichtdurchlässigen Bereiche (42a, 42b, 42c) der Maske (37).

12. Sehprüfgerät nach einem der Ansprüche 4 bis 11, **dadurch gekennzeichnet, dass** die Breite ($B_D$) der streifenförmigen Zonen (30a, 30b, 30c, 34a, 34b, 34c) des Displays (14) einem Durchmesser P eines Displaypixels (16) für das Erzeugen eines Bildpunkts entspricht.

13. Untersuchungseinrichtung mit einem gemäß einem der Ansprüche 1 bis 12 ausgebildeten Sehprüfgerät und mit einem Probandenbereich, in dem ein Proband (6, 106) für das Durchführen einer Sehprüfung in einem Abstand A von dem Display (14, 114) des Sehprüfgeräts (2, 102) positioniert werden kann, für den gilt: 1 m ≤ A ≤ 7m, vorzugsweise 2m ≤ A ≤ 3m.

14. Verwendung eines Sehprüfgeräts (2, 102) gemäß einem der Ansprüche 1 bis 12 oder einer Untersuchungseinrichtung (1, 101) gemäß Anspruch 13 für das Untersuchen der Winkelfehlsichtigkeit der Augen (12a, 12b, 112a, 112b) eines Probanden (6, 106), insbesondere für das Durchführen des Kreuztests oder des Zeigertests oder des senkrechten Rechtecktest oder des Dreieckstests oder des Stereo-Sehgleichgewichtstests oder des Doppelzeigertests.

15. Verfahren für das Untersuchen von Winkelfehlsichtigkeiten der Augen eines Probanden mit einer gemäß einem der Ansprüche 1 bis 12 ausgebildeten Sehprüfgerät (2, 102) oder mit einer gemäß Anspruch 13 ausgebildeten Untersuchungseinrichtung (1, 101), bei welchem mit der ersten und der zweiten Gruppe (28, 128, 32, 134) ausgewählter Bereiche des Displays (13, 114) zwei wenigstens abschnittsweise zueinander komplementäre Teilmuster (46, 50) insbesondere für das Durchführen des Kreuztests oder des Rechtecktests oder des Dreieckstests oder des Stereo-Sehgleichgewichtstests erzeugt werden, wobei eines der beiden Teilmuster dem linken Auge und das andere Teilmuster dem rechten Auge des Probanden zur Anzeige gebracht wird.

## Claims

1. Eyesight testing device (102) for examining associated heterophoria of the eyes (112a, 112b) of a subject (106), having an image generation apparatus (108), which has a display unit (114) for producing test patterns, which can be displayed to the eyes (112a, 112b) of the subject (106) with an optical beam path (122), wherein, on that side of the display unit (114) which faces the eyes (112a, 112b) of the subject in the optical beam path (122), an optical assembly (124) is arranged, which separates the light supplied by a first group (128) of selected zones (130a, 130b, 130c) of the display unit (114) to the optical beam path (122) from the light which is supplied to the beam path (122) by a second group (132) of selected zones (134a, 134b, 134c) of the display unit (114), in order to supply the light from the first group (128) of selected zones (130a, 130b, 130c) of the display unit (114) to the left eye (112a) of the subject (106) and to guide the light from the second group (132) of selected zones (134a, 134b, 134c) of the display unit to the right eye (112b) of the subject (106), **characterized in that** the optical assembly (124) comprises a prism matrix (125), which has a multiplicity of prism portions (127) which extend in the vertical direction (139) and have in each case a lens-shaped, in particular cylindrical-lens-shaped region (129) with a convex surface facing the display unit (114).

2. Eyesight testing device according to Claim 1, **characterized in that** the display unit (114) has a multiplicity of pixels (16) for producing image points in mutually complementary zones (130a, 130b, 130c, ...; 134a, 134b, 134c, ...) of the display unit (114), which extend in the vertical direction (139), and the lens-shaped region (129) of the prism portions (127) facing the display unit (114) is cylindrical-lens-shaped and has a cylindrical lens axis extending in the vertical direction (139).

3. Eyesight testing device according to Claim 1 or 2, **characterized in that** the optical assembly (124) is adjustable for defining the course of the optical beam path (122) to the eyes (112a, 112b) of the subject (106).

4. Eyesight testing device (2) for examining associated heterophoria of the eyes (12a, 12b) of a subject (6), having an image generation apparatus (8), which has a display unit (14) for producing test patterns, which can be displayed to the eyes (12a, 12b) of the subject (6) with a optical beam path (22), wherein, on that side (26) of the display unit (14) which faces the eyes (12a, 12b) of the subject in the optical beam path (22), an optical assembly (24) is arranged, which separates the light supplied by a first group (28) of selected zones (30a, 30b, 30c, 30d) of the display unit (14) to the optical beam path (22) from the light which is supplied to the beam path (22) by a second group (32) of selected zones (34a, 34b, 34c, 34d) of the display unit (14), in order to supply the light from the first group (28) of selected zones (30a, 30b, 30c, 30d) of the display unit (14) to the left eye (12a) of the subject (6) and to guide the light from the second group (32) of selected zones (34a, 34b, 34c, 34d) of the display unit to the right eye (12b) of the subject (6),

**characterized in that**

the optical assembly (24) contains a screen system (36) acting as a parallax barrier for separating the light from the first and second groups (28, 32) of selected zones (30a, 30b, 30c, 30d) of the display unit (14), which screen system comprises a mask (37, 89) with alternately opaque and light-transmissive regions (40a, 40b, 40c, 42a, 42b, 42c; 90a, 90b, 90c, ...; 92a, 92b, 92c; ...), and

the display unit (14) has a multiplicity of pixels (16) for producing image points in mutually complementary zones (30a, 30b, 30c, ...; 34a, 34b, 34c, ...) of the display unit (14), which extend in the vertical direction (39), wherein

the mutually complementary zones (30a, 30b, 30c, ...; 34a, 34b, 34c, ...) of the display unit (14) are stripe-shaped and the width ($B_{Mn}$) of the regions (40a, 40b, 40c, ...) of the mask (37) which are opaque for light from the display unit (14) and the width ($B_{Md}$) of the regions (42a, 42b, 42c, ...) of the mask (37) which are transmissive for light from the display unit (14) and the width ($B_D$) of the stripe-shaped zones (30a, 30b, 30c, 34a, 34b, 34c) of the display unit (14) satisfy the following relationship:

$$B_{Mn} \approx 3\ B_{Md} \approx 3/2\ B_D,$$

wherein the distance z of the mask (37) from the display unit (14) corresponds to 25 to 50 times the width $B_D$ of the zones (30a, 30b, 30c, 34a, 34b, 34c) of the display unit (14).

**5.** Eyesight testing device according to Claim 4, **characterized in that** the optical assembly (24) is adjustable for defining the course of the optical beam path (22) to the eyes (12a, 12b) of the subject (6).

**6.** Eyesight testing device according to one of Claims 4 and 5, **characterized in that** the width ($B_D$) of the stripe-shaped zones (30a, 30b, 30c) of the display unit (14) and the width ($B_{Md}$, $B_{Mu}$) of the regions (100a, 100b, ...; 98a, 98b, 98c, ...) of the mask (96) which are transmissive or opaque for the light from the display unit (14) is adjustable.

**7.** Eyesight testing device according to one of Claims 4 to 6, **characterized in that** the width ($B_D$) of the stripe-shaped zones (30a, 30b, 30c, 34a, 34b, 34c) of the display unit (14) corresponds to a diameter P of a display-unit pixel (16) for producing an image point.

**8.** Eyesight testing device according to one of Claims 4 to 7, **characterized in that** the screen system (36) is moveable perpendicular and/or parallel relative to the display unit (14), wherein at least the distance (z) between a light passage plane (41) of the screen system (36), which separates the light for the left and right eyes and the display unit (14), and/or a position of the alternately light-transmissive and opaque regions (40a, 40b, 40c, 42a, 42b, 42c) in a light passage plane (41) of the screen system (36) can be varied.

**9.** Eyesight testing device according to Claim 8, **characterized by** a device (25, 20) for capturing the angular position of the eyes (12a, 12b) of a subject (6), which is coupled to a drive (49) which shifts the screen system (36) on the basis of a captured angular position of the eyes (12a, 12b) of the subject (6) such that the deviation S of the centre (23) of the perpendicular projection of the pupillary distance (PD) of the eyes (12, 12b) of the subject (6) from a vertical line (39) in the middle of the mask (37) before the shift and a vertical displacement V of the middle of the mask (37) after the shift satisfies the following relationship:

$$\frac{V}{z} = \frac{S}{g_0},$$

wherein z is the distance of the light passage plane 41 of the screen system 36 from the display unit 14 and $g_0$ is the distance of the subject from the light passage plane 41 of the screen system 36.

**10.** Eyesight testing device according to one of Claims 4 to 9, **characterized in that** the display unit (14) has a multiplicity of pixels (16) for producing image points in mutually complementary zones (30a, 30b, 30c, ...; 34a, 34b, 34c, ...) of the display unit (14), which extend in the vertical direction (39).

**11.** Eyesight testing device according to Claim 10, **characterized in that** the horizontal width ($B_{Mn}$) of the opaque regions (40a, 40b, 40c, ...) of the mask (37) is at least twice the width ($B_{Md}$) of the light-transmissive regions (42a, 42b, 42c) of the mask (37).

**12.** Eyesight testing device according to one of Claims 4 to 11, **characterized in that** the width ($B_D$) of the stripe-shaped zones (30a, 30b, 30c, 34a, 34b, 34c) of the display unit (14) corresponds to a diameter P of a display-unit pixel (16) for producing an image point.

13. Examination device with an eyesight testing device configured according to one of Claims 1 to 12 and with a subject region, in which, for performing an eyesight test, a subject (6, 106) can be positioned at a distance A from the display unit (14, 114) of the eyesight testing device (2, 102), for which: 1m ≤ A ≤ 7m, preferably 2m ≤ A ≤ 3m.

14. Use of an eyesight testing device (2, 102) according to one of Claims 1 to 12 or of an examination device (1, 101) according to Claim 13 for examining associated heterophoria of the eyes (12a, 12b, 112a, 112b) of a subject (6, 106), in particular for carrying out the cross test or the pointer test or the perpendicular rectangle test or the triangle test or the stereo balance test or of the double pointer test.

15. Method for examining associated heterophoria of the eyes of a subject with an eyesight testing device (2, 102) configured according to one of Claims 1 to 12 or with an examination device (1, 101) configured according to Claim 13, in which, with the first and the second groups (28, 128, 32, 134) of selected regions of the display unit (13, 114), two partial patterns (46, 50), which are at least in portions mutually complementary, are produced in particular for carrying out the cross test or the rectangle test or the triangle test or the stereo balance test, wherein one of the two partial patterns is displayed to the left eye and the other partial pattern is displayed to the right eye of the subject.

## Revendications

1. Appareil de test de la vision (102) destiné à examiner l'hétérophorie avec disparité de fixation des yeux (112a, 112b) d'un sujet (106),

comportant un dispositif de formation d'image (108) comprenant un afficheur (114) destiné à générer des motifs de test qui peuvent être présentés aux yeux (112a, 112b) du sujet (106) suivant un chemin optique (122),

dans lequel un montage optique (124) est disposé du côté de l'afficheur (114) qui est tournée vers les yeux (112a, 112b) du sujet, sur le chemin optique (122), lequel montage sépare la lumière acheminée par un premier groupe (128) de zones sélectionnées (130a, 130b, 130c) de l'afficheur (114) vers le chemin optique (122) de la lumière qui est acheminée vers le chemin optique (122) par un second groupe (132) de zones sélectionnées (134a, 134b, 134c) de l'afficheur (114) afin d'acheminer la lumière provenant du premier groupe (128) de zones sélectionnées (130a, 130b, 130c) de l'afficheur (114) vers l'oeil gauche (112a) du sujet (106) et de diriger la lumière provenant du second groupe (132) de zones sélectionnées (134a, 134b, 134c) de l'afficheur vers l'oeil droit (112b) du sujet (106), **caractérisé en ce que**

le montage optique (124) comprend une matrice de prismes (125) comportant une pluralité de sections de prismes (127) s'étendant en direction verticale (139), ayant respectivement une région (129) en forme de lentille opposée à l'afficheur (114), notamment de lentille cylindrique ayant une surface convexe.

2. Appareil de test de la vision selon la revendication 1, **caractérisé en ce que** l'afficheur (114) comprend une pluralité de pixels (16) pour générer des points d'image dans des zones mutuellement complémentaires (130a, 130b, 130c, ... ; 134a, 134b, 134c, ...) de l'afficheur (114), qui s'étendent en direction verticale (139) et la région (129) en forme de lentille tournée vers l'afficheur (114) des sections de prismes (127) présente la forme d'une lentille cylindrique et a un axe de lentille cylindrique s'étendant dans la direction verticale (139).

3. Appareil de test de la vision selon la revendication 1 ou 2, **caractérisé en ce que** le montage optique (124) peut être réglé pour établir le parcours du chemin optique (122) vers les yeux (112a, 112b) du sujet (106).

4. Appareil de test de la vision (2) destiné à analyser l'hétérophorie avec disparité de fixation des yeux (12a, 12b) d'un sujet (6),

comportant un dispositif de formation d'image (8) comprenant un afficheur (14) destiné à générer des motifs de test qui peuvent être présentés aux yeux (12a, 12b) du sujet (6) suivant un chemin optique (22), dans lequel un montage optique (24) est disposé du côté (26) de l'afficheur (14) qui est tourné vers les yeux (12a, 12b) du sujet, sur le chemin optique (22), lequel montage sépare la lumière acheminée par un premier groupe (28) de zones sélectionnées (30a, 30b, 30c, 30d) de l'afficheur (14) vers le chemin optique (22) de la lumière qui est acheminée vers le chemin optique (22) par un second groupe (32) de zones sélectionnées (34a, 34b, 34c, 34d) de l'afficheur (14) afin d'acheminer la lumière provenant du premier groupe (28) de zones sélectionnées (30a, 30b, 30c, 30d) de l'afficheur (14) vers l'oeil gauche (12a) du sujet (6) et de diriger la lumière provenant du second groupe (32) de zones sélectionnées (34a, 34b, 34c, 34d) de l'afficheur vers l'oeil droit (12b) du sujet (6),

**caractérisé en ce que**

le montage optique (24) contient un système de diaphragme (36) fonctionnant en tant que barrière de parallaxe pour séparer la lumière des premier et second groupes (28, 32) de zones sélectionnées (30a, 30b, 30c, 30d) de l'afficheur (14), qui comprend un masque (37, 89) comportant des régions alternativement opaques et transparentes (40a, 40b, 40c, 42a, 42b, 42c ; 90a, 90b, 90c, ... ; 92a, 92b, 92c ; ...), et

l'afficheur (14) comprend une pluralité de pixels (16) pour générer des points d'image dans des zones mutuellement complémentaires (30a, 30b, 30c, ... ; 34a, 34b, 34c, ...) de l'afficheur (14) s'étendant en direction verticale (39), dans lequel

les zones mutuellement complémentaires (30a, 30b, 30c, ... ; 34a, 34b, 34c, ...) de l'afficheur (14) présentent la forme de bandes et la largeur ($B_{Mn}$) des régions du masque (37) opaques (40a, 40b, 40c, ...) à la lumière de l'afficheur (14) et la largeur ($B_{Md}$) des régions du masque (37) transparentes (42a, 42b, 42c, ...) à la lumière de l'afficheur (14), ainsi que la largeur ($B_D$) des zones en forme de bandes (30a, 30b, 30c, 34a, 34b, 34c) de l'afficheur (14) satisfont à la relation suivante :

$$B_{Mn} \approx 3 \; B_{Md} \approx 3/2 \; B_D$$

dans lequel la distance z du masque (37) à l'afficheur (14) correspond à 25 à 50 fois la largeur ($B_D$) des zones (30a, 30b, 30c, 34a, 34b, 34c) de l'afficheur (14).

5. Appareil de test de la vision selon la revendication 4, **caractérisé en ce que** le montage optique (24) peut être réglé pour établir le parcours du chemin optique (22) vers les yeux (12a, 12b) du sujet (6).

6. Appareil de test de la vision selon la revendication 4 ou 5, **caractérisé en ce que** la largeur ($B_D$) des zones en forme de bandes (30a, 30b, 30c) de l'afficheur (14) et les largeurs ($B_{Md}$, $B_{Mu}$) des régions du masque (96) transparentes et opaques (100a, 100b, ... ; 98a, 98b, 98c, ...) à la lumière de l'afficheur (14) peuvent être réglées.

7. Appareil de test de la vision selon l'une quelconque des revendications 4 à 6, **caractérisé en ce que** la largeur ($B_D$) des zones en forme de bandes (30a, 30b, 30c, 34a, 34b, 34c) de l'afficheur (14) correspond à un diamètre P d'un pixel d'affichage (16) destiné à générer un point d'image.

8. Appareil de test de la vision selon l'une quelconque des revendications 4 à 7, **caractérisé en ce que** le système de diaphragme (36) est mobile perpendiculairement et/ou parallèlement à l'afficheur (14), dans lequel on peut faire varier la distance (z) entre un plan optiquement transmissif (41) du système de diaphragme (36) séparant la lumière destinée aux yeux gauche et droit, et l'afficheur (14) et/ou une position des régions alternativement opaques et transparentes (40a, 40b, 40c, 42a, 42b, 42c) dans un plan optiquement transmissif (41) du système de diaphragme (36).

9. Appareil de test de la vision selon la revendication 8, **caractérisé par** un dispositif (25, 20) destiné à détecter la position angulaire des yeux (12a, 12b) d'un sujet (6), lequel dispositif est couplé à un système d'entraînement (49) qui déplace le système de diaphragme (36) sur la base d'une position angulaire détectée des yeux (12a, 12b) du sujet (6) de manière à ce que l'écart S entre le centre (23) de la projection verticale de la distance interpupillaire (PD) des yeux (12, 12b) du sujet (6) et une droite verticale (39) au milieu du masque (37) avant le déplacement et un décalage vertical V du milieu du masque (37) après le déplacement, satisfait à la relation suivante :

$$\frac{V}{z} = \frac{S}{g_0},$$

où z est la distance du plan optiquement transmissif (41) du système de diaphragme (36) de l'afficheur (14) et $g_0$ est la distance du sujet au plan optiquement transmissif (41) du système de diaphragme (36).

10. Appareil de test de la vision selon l'une quelconque des revendications 4 à 9, **caractérisé en ce que** l'afficheur (14) comprend une pluralité de pixels (16) pour générer des points d'image dans des zones mutuellement complémentaires (30a, 30b, 30c, ... ; 34a, 34b, 34c, ...) de l'afficheur (14), qui s'étendent en direction verticale (39).

11. Appareil de test de la vision selon la revendication 10, **caractérisé en ce que** la largeur horizontale ($B_{Mn}$) des régions opaques à la lumière (40a, 40b, 40c, ...) du masque (37) est au moins deux fois supérieure à la largeur ($B_{Md}$) des régions transparentes à la lumière (42a, 42b, 42c) du masque (37).

12. Appareil de test de la vision selon l'une quelconque des revendications 4 à 11, **caractérisé en ce que** la largeur ($B_D$) des zones en forme de bandes (30a, 30b, 30c, 34a, 34b, 34c) de l'afficheur (14) correspond à un diamètre P d'un pixel d'affichage (16) destiné à générer un point d'image.

13. Dispositif d'inspection comportant un appareil de test de la vision conçu selon l'une quelconque des revendications 1 à 12 et comportant une région de sujet dans laquelle un sujet (6, 106) devant être soumis à un test de la vision peut être positionné à une distance A de l'afficheur (14, 114) de l'appareil de test de la vision (2, 102), avec 1 m ≤ A ≤ 7 m, et de préférence 2 m ≤ A ≤ 3 m.

14. Utilisation d'un appareil de test de la vision (2, 102) selon l'une quelconque des revendications 1 à 12, ou d'un dispositif d'inspection (1, 101) selon la revendication 13 pour l'examen de l'hétérophorie avec disparité de fixation des yeux (12a, 12b, 112a, 112b) d'un sujet (6, 106), notamment pour la réalisation du test de la croix, ou du test de l'aiguille ou du test du carré vertical ou du test du triangle ou du test d'équilibrage de la stéréovision ou du test de la double aiguille.

15. Procédé d'inspection de l'hétérophorie avec disparité de fixation des yeux d'un sujet, comportant un appareil de test de la vision (2, 102) conçu selon l'une quelconque des revendications 1 à 12 ou comportant un dispositif d'inspection (1, 101) conçu selon la revendication 13, dans lequel, au moyen des premier et deuxième groupes (28, 128, 32, 134) de régions sélectionnées de l'afficheur (13, 114), deux motifs partiels (46, 50) mutuellement complémentaires au moins par sections sont générés, notamment pour la réalisation du test de la croix ou du test du carré ou du test du triangle ou du test d'équilibrage de la stéréovision, dans lequel l'un des deux motifs partiels est présenté à l'oeil gauche et l'autre motif partiel est présenté à l'oeil droit du sujet.

Fig.1

Fig.2

Fig.3

Fig.4

Fig.5

EP 2 606 815 B1

Fig.6a

Fig.6b

Fig.7a

Fig.7b

Fig.7c

Fig.7d

Fig.8

Fig.9

Fig.10

EP 2 606 815 B1

Fig.11

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0512443 B1
- US 5877840 A